Europäisches Patentamt

European Patent Office

Office européen des brevets

(19)

(11) **EP 0 691 830 B1**

(12) **EUROPÄISCHE PATENTSCHRIFT**

(45) Veröffentlichungstag und Bekanntmachung des Hinweises auf die Patenterteilung:
**25.08.1999 Patentblatt 1999/34**

(21) Anmeldenummer: **94908314.1**

(22) Anmeldetag: **16.02.1994**

(51) Int. Cl.$^6$: **A61F 2/38**

(86) Internationale Anmeldenummer:
**PCT/EP94/00434**

(87) Internationale Veröffentlichungsnummer:
**WO 94/22396** (13.10.1994 Gazette 1994/23)

(54) **KÜNSTLICHES GELENK ZUM ERSATZ DER MENSCHLICHEN KNIESCHEIBE**

ARTIFICIAL JOINT TO REPLACE THE HUMAN PATELLA

ARTICULATION ARTIFICIELLE PERMETTANT DE REMPLACER LA ROTULE CHEZ L'HOMME

(84) Benannte Vertragsstaaten:
**AT BE CH DE DK ES FR GB GR IE IT LI LU MC NL PT SE**

(30) Priorität: **03.04.1993 DE 4310968**

(43) Veröffentlichungstag der Anmeldung:
**17.01.1996 Patentblatt 1996/03**

(73) Patentinhaber:
• **Theusner, Joachim, Dr.**
  **80539 München (DE)**
• **Kubein-Meesenburg, Dietmar, Prof. Dr.**
  **37547 Kreiensen (DE)**
• **Nägerl, Hans, Dr.**
  **37130 Gleichen (DE)**

(72) Erfinder:
• **KUBEIN-MEESENBURG, Dietmar, Prof. Dr.**
  **D-37547 Kreiensen (DE)**
• **NÄGERL, Hans, Dr.**
  **D-37130 Gleichen/OT Kelin-Lengden (DE)**

(74) Vertreter:
**Braun, Dieter, Dipl.-Ing. et al**
**Hagemann, Braun & Held**
**Patentanwälte,**
**Hildesheimer Strasse 133**
**30173 Hannover (DE)**

(56) Entgegenhaltungen:
**WO-A-90/11062**          **WO-A-93/11720**
**US-A- 3 964 106**        **US-A- 4 081 866**
**US-A- 4 158 894**        **US-E- R E29 757**

## Beschreibung

Die vorliegende Erfindung betrifft ein künstliches Gelenk als Endoprothese für das menschliche Kniescheiben-Gelenk.

Die US-Patentschrift US-A-3 964 106 offenbart ein Künstliches Gelenk als Endoprothese für das menschliche Kniescheiben-Gelenk, bestehend aus zwei zueinander sich bewegenden Gelenkkörpern, einem das Oberschenkel-Gelenkteil bildenden Gelenkkörper und einem das Patella-Gelenkteil bildenden Gelenkkörper.

Aus der deutschen Patentanmeldung DE-A-39 08 958, zu deren Patentfamilie auch die WO-A-9011062 gehört, ist ein künstliches Gelenk zum Ersatz von menschlichen Gelenken bekannt, bestehend aus mindestens zwei Gelenkteilen mit zueinander sich bewegenden, sphärischen Funktionsflächen. Die Krümmungsverhältnisse der eine kreisförmige Schnittkontur aufweisenden Funktionsflächen sind zueinander konvex-konvex, konvex-konkav oder konkav-konkav, und die Gelenkgeometrie ist durch eine Gelenkkette mit zwei Gelenkachsen, dimere Gelenkkette, bestimmt, die durch die Rotationszentren der Funktionsflächen verlaufen und durch die Zentren und deren Abstand definiert wird. Hierbei sind die Gelenkflächen kugelförmig ausgebildet, so daß eine Gelenkbewegung mit fünf Freiheitsgraden möglich ist.

Es hat sich jedoch gezeigt, daß ein derartiges Gelenk nicht geeignet ist, um die spezielle Gelenkfunktion, wie sie bei dem menschlichen Kniescheibengelenk vorhanden ist, nachzubilden.

Der vorliegenden Erfindung liegt die Aufgabe zugrunde, ein künstliches Gelenk zu schaffen, das geeignet ist zum Ersatz des menschlichen kniescheibengelenks, wobei die natürlichen Verhältnisse im wesentlichen nachgebildet werden.

Erfindungsgemäß wird dies durch ein künstliches Gelenk gemäß den Merkmalen des Anspruchs 1 erreicht.

Die Erfindung beruht somit auf der Erkenntnis, daß die Gelenkbahnen des menschlichen kniescheibengelenks durch jeweils toroidförmige Flächen der Schnittkonturen in den zueinander senkrechten Ebenen ersetzt werden können.

Die hierbei auftretenden Druckbeanspruchungen können durch die Verwendung entsprechend fester Materialien beherrscht werden. Es wird somit ein künstliches Gelenk geschaffen, das eine besondere Bewegungsfreiheit in einer Gelenkebene besitzt, und das gleichzeitig eine hohe mechanische Stabilität mit einer großen Variationsbreite zur Anpassung an die individuellen Gegebenheiten aufweist sowie zusätzlich eine leichte Beweglichkeit in den zur Längsebene senkrechten Querebenen nach lateral aufgrund spezieller Konstruktionsmerkmale.

Vorteilhafte Ausführungen der Erfindung sind in den Unteransprüchen enthalten.

Anhand der in den beiliegenden Zeichnungen dargestellten Ausführungsbeispiele wird die Erfindung näher erläutert. Es zeigen:

Fig. 1    eine perspektivische Ansicht eines erfindungsgemäßen Gelenkkörpers mit zwei zusammengefaßten Gelenkköpfen,

Fig. 2    eine perspektivische Ansicht eines erfindungsgemäßen Gelenkkörpers mit zwei zusammengefaßten Gelenkpfannen,

Fig. 3    einen Längsschnitt durch ein erfindungsgemäßes Gelenk im Bereich des lateralen Gelenkteils,

Fig. 4    einen Querschnitt gemäß der Schnittlinie IV-IV in Fig. 3 in der Querebene,

Fig. 5    einen Längsschnitt durch ein erfindungsgemäßes Gelenk im medialen Gelenkteil,

Fig. 6    einen Schnitt entlang der Schnittlinie VI-VI in Fig. 5 in der Querebene,

Fig. 7    einen Längsschnitt durch ein erfindungsgemäßes Gelenk, zusammengesetzt aus den Gelenken gemäß Fig. 3 und 5.

Fig. 8    eine perspektivische Ansicht einer weiteren Ausführungsform eines erfindungsgemäßen Gelenkkörpers mit zwei zusammengefaßten Gelenkpfannen.

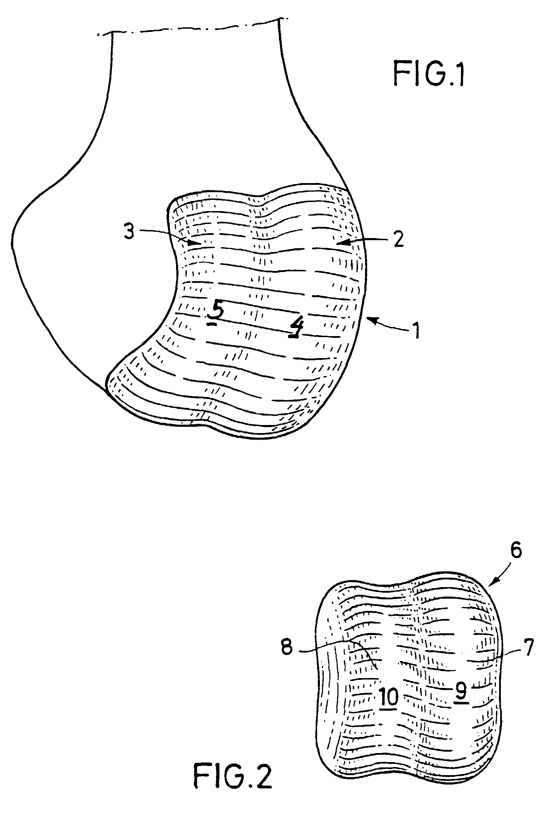

In Fig. 1 ist eine perspektivische Darstellung eines Teils des Oberschenkels, Femur, gezeigt, mit an dessen Gelenkkörper befestigten Oberschenkelgelenkteil 1, das aus zwei nebeneinanderliegenden Gelenkköpfen besteht, und zwar einem lateralen Gelenkkopf 2 und einem medialen Gelenkkopf 3. Die beiden Gelenkköpfe 2, 3 besitzen toroidförmig ausgebildete Funktionsflächen 4, 5, und zwar die laterale Funktionsfläche 4 und die mediale Funktionsfläche 5, wie sich dies im einzelnen aus der nachfolgenden Beschreibung ergibt.

Fig. 2 zeigt in perspektivischer Ansicht das zum Oberschenkelgelenkteil 1 zugehörige Patella-Gelenkteil 6, das aus zwei zusammengefaßten Gelenkpfannen besteht, und zwar einer medialen Gelenkpfanne 7 und einer lateralen Gelenkpfanne 8. Die beiden Gelenkpfannen 7, 8 besitzen wiederum toroidförmig gekrümmt ausgebildete Funktionsflächen 9, 10 und zwar die mediale Funktionsfläche 9 und die laterale Funktionsfläche 10. Die nähere Ausgestaltung ergibt sich aus der folgenden Beschreibung. Der laterale Gelenkkopf 2 und die laterale Gelenkpfanne 8 bilden ein erfindungsgemäßes Lateral-Gelenk und die mediale Gelenkpfanne 7 und der mediale Gelenkkopf 3 bilden ein erfindungsgemäßes Medial-Gelenk.

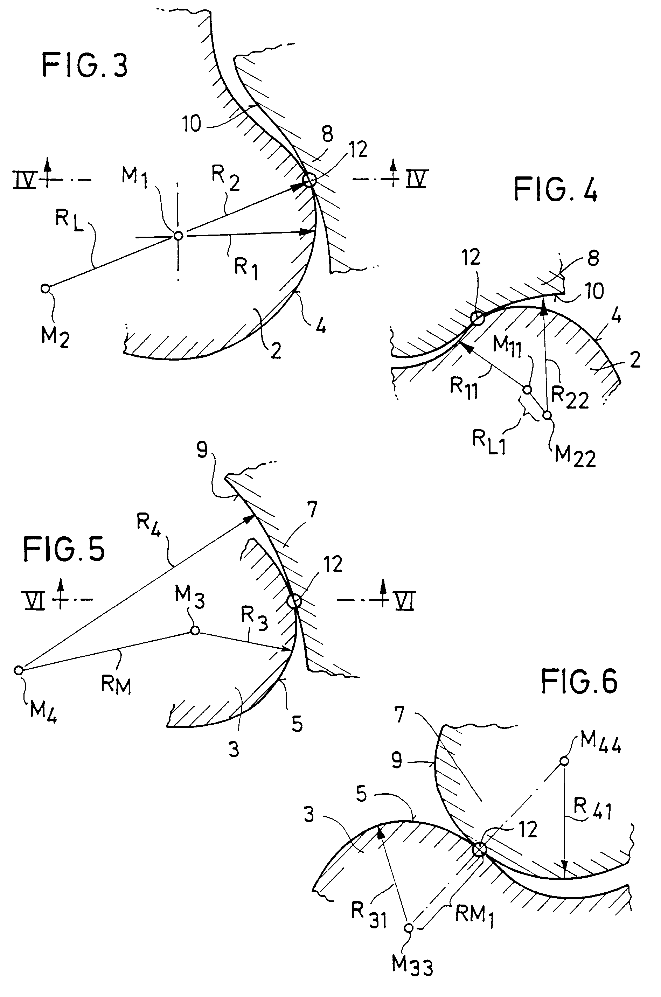

Wie sich aus Fig. 3 ergibt, besitzt die laterale Funktionsfläche 4 des lateralen Gelenkkopfes 2 in der sagitalen Schnittebene, d.h. in der Längsebene, eine kreisbogenförmige, konvexe Schnittkontur, deren Rotationszentrum $M_1$ ist und die den Radius $R_1$ besitzt. Die laterale Gelenkpfanne 8 besitzt eine Funktionsfläche 10 mit einer kreisförmigen, konkaven Schnittkontur mit dem Rotationszentrum $M_2$ und dem Radius $R_2$. Hierbei ist eine derartige Anordnung vorgesehen, daß diese Rotationszentren $M_1$ und $M_2$ innerhalb des Gelenkteils mit der konvexen Schnittkontur liegen und die Gelenkachsenbahnen der Rotationszentren einen Radius $RL = R_2 - R_1$ besitzen. Hierbei ist $R_2$ derart bemessen, daß $R_2$ größer ist als $R_1$, somit stellt diese Anordnung eine überschlagene, druckstabile dimere Gelenkkette dar.

In Fig. 4 ist zu erkennen, daß auch in der Querebene die lateralen Funktionsflächen 4, 10 kreisförmige Schnittkonturen besitzen, wobei die kreisförmige konvexe Schnittkontur der Funktionsfläche 4 den Radius $R_{11}$ und den Mittelpunkt bzw. das Rotationszentrum $M_{11}$ besitzt und die kreisförmige, konkave Funktionsfläche 10 den Radius $R_{22}$ sowie den Mittelpunkt $M_{22}$ aufweist. Hierbei liegen beide Rotationszentren $M_{11}$ und $M_{22}$ im Körper mit der konvexen Funktionsfläche 4 und die Gelenkachsenbahn der Rotationszentren hat einen Radius $RL_1 = R_{22} - R_{11}$, wobei $R_{22}$ größer ist als $R_{11}$, so daß diese Anordnung eine überschlagene, druckstabile dimere Gelenkkette darstellt. Weiterhin ist in den Fig. 3 und 4 zu erkennen, daß die Mittelpunkte $M_{11}$ und $M_{22}$ nicht mit den Rotationszentren $M_1$ bzw. $M_2$ zusammenfallen, was vorteilhaft ist. $M_2$ liegt vorteilhafterweise in der Position des gestreckten Beines gegenüber $M_1$ nach hinten (kaudal) versetzt, während $M_{22}$ in bezug auf $M_{11}$ nach außen versetzt ist.

In Fig. 5 ist wiederum ein Schnitt durch die Längsebene bzw. in der sagitalen Ebene des erfindungsgemäßen Medial-Gelenks dargestellt. Der mediale Gelenkkopf 3 besitzt die Funktionsfläche 5, die toroidförmig ausgebildet ist und eine kreisförmige, konvexe Schnittkontur besitzt, wobei diese kreisförmige Schnittkontur den Mittelpunkt bzw. das Rotationszentrum $M_3$ und den Radius $R_3$ besitzt. Die mediale Gelenkpfanne 7 besitzt eine Funktionsfläche 9, die in der Längsebene eine kreisförmige, konkave Schnittkontur aufweist, die den Mittelpunkt $M_4$ und den Radius $R_4$ besitzt. Wie dargestellt ist, liegen die Rotationszentren $M_3$ und $M_4$ jeweils im Körper mit der konvexen Schnittkontur der Funktionsfläche und die Gelenkachsenbahn der Rotationszentren $M_3$ und $M_4$ besitzt einen Radius $RM = R_4 - R_3$, wobei $R_4 > R_3$ ist, so daß sich eine druckstabile, dimere Gelenkkette ergibt.

In Fig. 6 ist der Schnitt gemäß der Frontalebene, Querebene, zu der Darstellung in Fig. 5 gezeigt. Hierbei ist zu erkennen, daß auch in dieser Schnittebene die Funktionsflächen 5, 9 jeweils kreisförmige Schnittkonturen besitzen. Die Funktionsfläche 5 des medialen Gelenkkopfes 3 weist dabei eine kreisförmige Schnittkontur mit dem Mittelpunkt $M_{33}$ mit dem Radius $R_{31}$ auf. Die Funktionsfläche 9 der medialen Gelenkpfanne besitzt in der Querebene eine kreisförmige, konvexe Schnittkontur mit dem Rotationszentrum $M_{44}$ und dem Radius $R_{41}$. Hierbei liegen die Rotationszentren $M_{33}$ und $M_{44}$ jeweils innerhalb des zugehörigen Gelenkkörpers 3, 7 und die Gelenkachsenbahn der Rotationszentren $M_{33}$ und $M_{44}$ besitzt einen Radius $RM_1 = R_{31} + R_{41}$.

Die Rotationszentren $M_3$ und $M_{33}$ müssen nicht zusammenfallen. Das Zentrum $M_4$ kann bezogen auf $M_3$ nach distal, hinten, und nach unten, kaudal, versetzt sein, wie das Rotationszentrum $M_{44}$, bezogen auf $M_{33}$ nach vorne und nach außen, lateral, versetzt sein kann. Das derart ausgebildete mediale Gelenkteil des erfindungsgemäßen Kniescheibengelenks soll die natürliche Artikulation zwischen dem inneren Gelenkteil des Oberschenkels (Femur) und dem inneren Anteil der Kniescheibe (Patella) ersetzen. Hierbei weist dieses Gelenk in der sagitalen Ebene (der Längsebene) eine überschlagene druckkraftschlüssige dimere Kette auf und in der oder den dazu senkrechten Querebenen eine nichtüberschlagene druckkraftschlüssige Gelenkkette. Wegen der toroidförmigen Ausformung der Gelenkflächen ist eine gute Bewegungsfreiheit unter Kraftschluß in der Längsebene und eine weitgehend eingeschränkte in der Querebene gegeben.

Wie sich aus den Fig. 1 und 2 ergibt, sind jeweils die medialen und lateralen Gelenkteile derartig miteinander verbunden, daß jeweils eine starre Verbindung zwischen den Gelenkköpfen und den Gelenkpfannen gegeben ist. Hierbei ist es vorteilhaft, daß in der oder in den Querebenen die konvexen Gelenkköpfe 2, 3 durch eine abgestimmte konkave Struktur verbunden sind und in der oder in den Querebenen die Gelenkpfannen 7, 8 durch eine abgestimmte konvexe Struktur verbunden sind. Hierbei ist es zweckmäßig, wenn der Radius der verbindenden konvexen Struktur zwischen den Gelenkköpfen 2, 3 nicht identisch ist mit den Radien $R_{44}$ und $R_{22}$. Der Radius, der die Gelenkpfannen 7, 8 verbindenden konvexen Struktur kann größer sein als der der konkaven verbindenden Struktur der Gelenkköpfe 2, 3. Durch die Kopplung der Gelenkköpfe und der Gelenkpfannen ist der mediale und der laterale Gelenkteil zueinander so angeordnet, daß die Drehachsen senkrecht zur Langsebene parallel zueinander verlaufen und so zueinander angeordnet sind, daß die medialen Drehachsen hinter den jeweiligen lateralen Drehachsen angeordnet sind und in der Längsebene als Funktionsrichtung erfindungsgemäß ein Gelenkviereck geschaffen wird. Die Drehachsen der toroidförmigen Flächen können auch schräg zueinander gestellt sein.

In der Ausführungform gemäß den Fig. 1 bis 6 sind die Radien $R_1$, $R_2$, $R_3$ und $R_4$ so gewählt, daß sie weitgehend harmonisch in die Führungsstrukturen des Kniegelenks übergehen. Hier ist weiterhin bei der Bemessung der Radien vorgesehen, daß sie gewährleisten, daß die Kontaktpunkte, die sich um $M_1$ bewegen, einen deutlich größeren Weg beschreiben als die Kontaktpunkte, die sich um $M_3$ bewegen. Hierbei stellen die Kontaktpunkte jeweils die Berührungspunkte der gegenüberliegenden Funktionsflächen dar. Weiterhin sind die Mittelpunkte $M_1$ und $M_2$ zueinander und die Mittelpunkte $M_2$ und $M_4$ zueinander genauso wie $M_2$ und $M_4$ zu $M_1$ und $M_2$ so gewählt, daß in der Startposition des Standes des menschlichen Knies die Kontaktpunkte im lateralen wie im medialen Gelenkteil weitgehend in einer nahezu horizontalen Querebene liegen. Mit zunehmender Beuge liegen die Kontaktpunkte in verschiedenen Querebenen und der Kontakt läuft auf dem lateralen Gelenkteil der Patella schneller nach kranial als auf den medialen Teil der Patella. Umgekehrt laufen die Kontaktpunkte mit zunehmender Beuge auf den medialen Gelenkkopfteil schneller nach kaudal, um in tiefer Beuge den Kontakt zu verlieren.

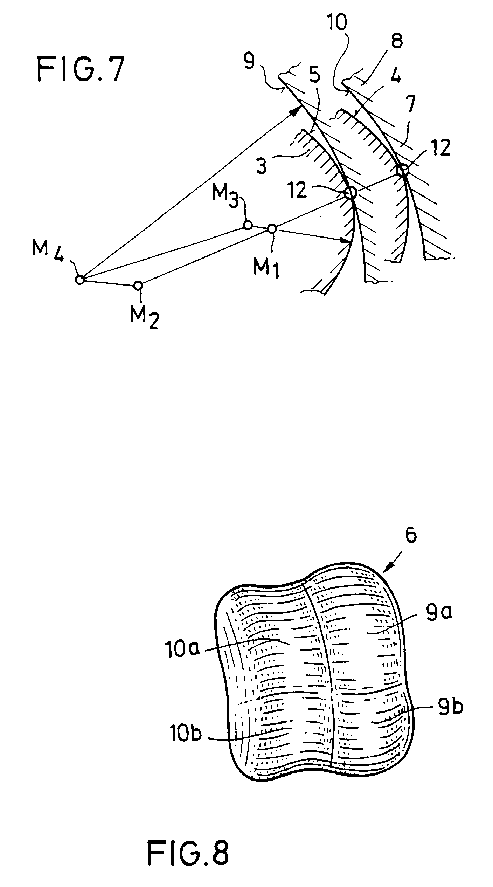

Fig. 7 zeigt einen Längsschnitt durch ein aus dem Medialgelenk und dem Lateralgelenk zusammengesetztes erfindungsgemäßes Gelenk, wobei das Medialgelenk hinter dem Lateralgelenk angeordnet ist. Gezeigt ist die Gelenkstellung zu Beginn einer Kniebeuge. Im übrigen sind gleiche Teile, wie in den Fig. 3 und 5, mit denselben Bezugsziffern versehen. Hierbei ist zu erkennen, daß das Lateralgelenk in der Längsebene gesehen nach vorne versetzt ist. Ebenfalls ist es möglich, das Lateralgelenk nach vorne und nach unten gegenüber dem Medialgelenk zu versetzen.

In Fig. 8 ist eine weitere Ausführungsform der Gelenkpfannen 9, 10 des Patella-Gelenkteils 6 dargestellt. Hierbei ist zu erkennen, daß die Funktionsfläche der medialen Gelenkpfanne und der lateralen Gelenkpfanne jeweils in zwei übereinanderliegende Teil-Funktionsflächen 9a, 9b und 10a, 10b unterteilt sind. Die grundsätzliche geometrische Form der Funktionsflächen 9a, 9b und 10a, 10b entspricht derjenigen der Funktionsflächen 9 und 10 gemäß den Fig. 2 bis 4. Hierbei ergibt sich ein buckelförmiger Übergang zwischen den Teilfunktionsflächen. Die Mittelpunkte der kreisförmigen Schnittkonturen der lateralen Funktionsflächen 10a, 10b liegen in derselben Ebene. Das gleiche gilt für die Mittelpunkte der medialen Funktionsflächen 9a und 9b. Sofern es sich um die kreisbogenförmige Schnittkontur in der Querebene der Funktionsflächen handelt, so tritt hier keine Änderung gegenüber der Ausführungsform in den Fig. 2 bis 6 auf. In den Darstellungen der Fig. 2 und 8 ist der Mittelgrad 12 zwischen den Funktionsflachen 9 und 10 bzw. 9a, 9b und 10a, 10b jeweils medial verbogen dargestellt, d.h. die jeweiligen Enden sind nach lateral versetzt. Es liegt ebenfalls im Rahmen der Erfindung, diesen Mittelgrad gradlinig verlaufend auszubilden.

Des weiteren liegt es im Rahmen der Erfindung, wenn in Abweichung des Ausführungsbeispiels der Fig. 6 die Funktionsfläche 9 in der Querebene eine konkave, kreisbogenförmige Schnittkontur besitzt, so daß sich eine druckstabile dimere Gelenkkette ausbildet, wobei der Radius der Gelenkachsenbahn $RM_1 = R_{41} - R_{31}$ ist, mit $R_{41} > R_{31}$, wobei die Mittelpunkte $M_{33}$ und $M_{44}$ im Gelenkkörper mit der konvexen Schnittkontur liegen. Eine entsprechende Ausbildung kann auch für die Funktionsflächen 9a, 9b, in Fig. 8 vorgesehen sein.

**Patentansprüche**

1. Künstliches Gelenk als Endoprothese für das menschliche Kniescheiben-Gelenk, bestehend aus zwei zueinander sich bewegenden Gelenkkörpern, einem das Oberschenkel-Gelenkteil bildenden Gelenkkörper (1) mit zwei zusammengefaßten nebeneinander liegenden Gelenkköpfen, und zwar einem lateralen Gelenkkopf (2) und einem medialen Gelenkkopf (3) und einem das Patella-Gelenkteil bildenden Gelenkkörper (6) mit zwei zusammengefaßten, nebeneinander liegenden Gelenkpfannen, und zwar eine lateralen Gelenkpfanne (8) und einer medialen Gelenkpfanne (7), die jeweils toroidförmige Funktionsflächen (4, 5; 9, 10) mit in zueinander senkrechten Ebenen - einer Längs - und einer entsprechenden Querebene - unterschiedlichen kreisförmigen Schnittkonturen besitzen, wobei die laterale Funktionsfläche (4) des lateralen Gelenkkopfes (2) in der Längsebene eine kreisbogenförmige, konvexe Schnittkontur mit dem Rotationszentrum $M_1$ und dem Radius $R_1$ besitzt und in der Längsebene gesehen das mediale Gelenk einen medialen Gelenkkopf (3) mit einer kreisförmigen, konvexen Schnittkontur mit dem Rotationszentrum $M_3$ und dem Radius $R_3$ besitzt und die laterale Gelenkpfanne (8) in der Längsebene gesehen eine Funktionsfläche (10) mit einer kreisförmigen, konkaven Schnittkontur mit dem Rotationszentrum $M_2$ und dem Radius $R_2$ aufweist und die mediale Gelenkpfanne (7) eine Funktionsfläche (9) mit einer in der Längsebene gesehen kreisförmigen, konkaven Schnittkontur mit dem Mittelpunkt $M_4$ und dem Radius $R_4$ besitzt, und die Radien $R_1$, $R_2$, $R_3$ und $R_4$ so gewählt sind, daß gewährleistet ist, daß die Kontaktpunkte, die sich um das Rotationszentrum $M_1$ bewegen, einen deutlich größeren Weg beschreiben als die Kontaktpunkte, die sich um das Rotationszentrum $M_3$ bewegen, wobei die Kontaktpunkte jeweils die Berührungspunkte der gegenüberliegenden Funktionsflächen darstellen und die Gelenkgeometrie der in Kontakt tretenden Funktionsflächen (4, 10; 5, 9) zueinander in jeder der beiden Funktionsebenen durch eine Gelenkkette mit zwei Gelenkachsen, dimere Gelenkkette, bestimmt ist, die durch die Krümmungsmittelpunkte $M_1$, $M_2$; $M_3$, $M_4$; $M_{11}$, $M_{22}$; $M_{33}$, $M_{44}$ der Funktionsflächen (4, 5; 9, 10) verlaufen und durch diese festgelegt sind.

2. Künstliches Gelenk nach Anspruch 1,

**dadurch gekennzeichnet,** daß die Rotationszentren $M_1$ und $M_2$ innerhalb des Gelenkteils mit der konvexen Schnittkontur liegen und die Gelenkachsenbahn der Rotationszentren einen Radius $RL = R_2 - R_1$ besitzen, wobei $R_2$ größer ist als $R_1$.

3. Künstliches Gelenk nach Anspruch 1 oder 2,
   **dadurch gekennzeichnet,**
   daß in der Querebene die laterale Funktionsfläche (4) des lateralen Gelenkkopfes (2) und die laterale Funktionsfläche (10) der Gelenkpfanne (8) eine kreisförmige Schnittkontur besitzen, wobei die kreisförmige, konvexe Schnittkontur der Funktionsfläche (4) den Radius $R_{11}$ und den Mittelpunkt $M_{11}$ besitzt und die kreisförmige, konkave Funktionsfläche (10) den Radius $R_{22}$ sowie den Mittelpunkt $M_{22}$ aufweist, und die Rotationszentren $M_{11}$ und $M_{22}$ im Körper mit der konvexen Funktionsfläche (4) liegen, und die Gelenkachsenbahn der Rotationszentren einen Radius $RL_1 = R_{22} - R_{11}$ besitzt, wobei $R_{22} > R_{11}$.

4. Künstliches Gelenk nach Anspruch 2 oder 3,
   **dadurch gekennzeichnet,**
   daß die Mittelpunkte $M_{11}$ und $M_{22}$ nicht mit den Rotationszentren $M_1$ bzw. $M_2$ zusammenfallen und $M_2$ vorteilhafterweise in der Position des gestreckten Beines gesehen gegenüber $M_1$ nach hinten versetzt ist, während $M_{22}$ in bezug auf $M_{11}$ nach außen versetzt ist.

5. Künstliches Gelenk nach einem der Ansprüche 1 bis 4, **dadurch gekennzeichnet,**
   daß die Rotationszentren $M_3$ und $M_4$ jeweils im Körper mit der konvexen Schnittkontur der Funktionsflächen liegen und die Gelenkachsenbahn der Rotationszentren $M_3$ und $M_4$ einen Radius $RM = R_4 - R_3$ aufweist, wobei $R_4 > R_3$ ist.

6. Künstliches Gelenk nach Anspruch 5,
   **dadurch gekennzeichnet,**
   daß im Schnitt durch die Querebene die Funktionsfläche (5) des Gelenkkopfes (3) und die Funktionsfläche (9) der Gelenkpfanne (7) jeweils kreisförmige Schnittkonturen aufweisen, wobei die Funktionsfläche (5) dabei eine konvexe Schnittkontur mit dem Mittelpunkt $M_{33}$ und dem Radius $R_{31}$ besitzt, und die Funktionsfläche (9) der medialen Gelenkpfanne eine kreisförmige, konvexe Schnittkontur mit dem Rotationszentrum $M_{44}$ und dem Radius $M_{41}$ besitzt, wobei die Rotationszentren $M_{33}$ und $M_{44}$ jeweils innerhalb des zugehörigen Gelenkkörpers (3, 7) sich befinden und die Gelenkachsenbahn der Rotationszentren $M_{33}$ und $M_{44}$ einen Radius $RM_1 = R_{31} + R_{41}$ besitzt.

7. Künstliches Gelenk nach einem der Ansprüche 1 bis 6, **dadurch gekennzeichnet,**

daß das Rotationszentrum $M_4$ bezogen auf das Rotationszentrum $M_3$ nach distal, d.h. nach hinten und nach unten, kaudal, versetzt ist sowie das Rotationszentrum $M_{44}$ bezogen auf das Rotationszentrum $M_{33}$ nach vorne und nach außen, lateral, versetzt ist.

8. Künstliches Gelenk nach einem der Ansprüche 1 bis 7, **dadurch gekennzeichnet,**
   daß in der oder in den Querebenen die konvexen Gelenkköpfe (2, 3) durch eine abgestimmte konkave Struktur verbunden sind und in der oder in den Querebenen die Gelenkpfannen (7, 8) durch eine abgestimmte konvexe Struktur verbunden sind, wobei vorteilhafterweise der Radius der verbindenden konkaven Struktur zwischen den Gelenkköpfen (2, 3) nicht identisch ist mit den Radien $R_{44}$ und $R_{22}$ und der Radius der die Gelenkpfannen (7, 8) verbindenden konvexen Struktur größer ist als der der konkaven verbindenden Struktur der Gelenkköpfe (2, 3).

9. Künstliches Gelenk nach einem der Ansprüche 1 bis 8, **dadurch gekennzeichnet,**
   daß die Mittelpunkte $M_1$ und $M_2$ zueinander und die Mittelpunkte $M_2$ und $M_4$ zueinander, ebenso wie die Mittelpunkte $M_2$ und $M_4$ zu den Mittelpunkten $M_1$ und $M_2$ so gewählt sind, daß in der Startposition des Standes des menschlichen Knies die Kontaktpunkte im lateralen wie im medialen Gelenkteil weitgehend in einer nahezu horizontalen Querebene liegen.

10. Künstliches Gelenk nach einem der Ansprüche 1 bis 9, **dadurch gekennzeichnet,**
    daß die Gelenkpfanne (9, 10) des Patella-Gelenkteils (6) Funktionsflächen aufweist, die jeweils in zwei übereinanderliegende Teil-Funktionsflächen (9a, 9b) und (10a, 10b) unterteilt sind, wobei die kreisförmige Schnittkontur dieser Teilfunktionsflächen derjenigen der entsprechenden Funktionsfläche (9, 10) entspricht.

11. Künstliches Gelenk nach einem der Ansprüche 1 bis 10, **dadurch gekennzeichnet,**
    daß die Funktionsfläche (9) in der Querebene gesehen eine konkave, kreisbogenförmige Schnittkontur besitzt, wobei der Radius der Gelenkachsenbahn $RM_1 = R_{41} - R_{31}$ ist mit $R_{41} > R_{31}$, wobei die Mittelpunkte $M_{33}$ und $M_{44}$ im Gelenkkörper mit der konvexen Schnittkontur liegen.

**Claims**

1. Artificial joint as endoprosthesis for the human patellar joint, consisting of two joint components which move in relation to one another, a joint component (1) forming the femoral joint part, having two joint heads combined and lying alongside one another, namely a lateral joint head (2) and a medial joint head (3), and a joint component (6) forming the patellar joint part, having two joint sockets combined and lying alongside one another, namely a lateral joint socket (8) and a medial joint socket (7), which each have toroidal functional surfaces (4, 5; 9, 10), respectively, with different circular sectional contours in mutually perpendicular planes, a longitudinal plane and a corresponding transverse plane, the lateral functional surface (4) of the lateral joint head (2) having in the longitudinal plane a circular arc-shaped, convex sectional contour with centre of rotation $M_1$ and radius $R_1$, and, viewed in the longitudinal plane, the medial joint having a medial joint head (3) with a circular, convex sectional contour with centre of rotation $M_3$ and radius $R_3$, and the lateral joint socket (8), viewed in the longitudinal plane, having a functional surface (10) with a circular, concave sectional contour with centre of rotation $M_2$ and radius $R_2$, and the medial joint socket (7) having a functional surface (9) with, viewed in the longitudinal plane, a circular, concave sectional contour with centre point $M_4$ and radius $R_4$, and the radii $R_1$, $R_2$, $R_3$ and $R_4$ are chosen in such a way as to ensure that the contact points which move about the centre of rotation $M_1$ describe a considerably greater path than the contact points which move about the centre of rotation $M_3$, the contact points in each case representing the points where opposite functional surfaces touch, and the joint geometry of the functional surfaces (4, 10; 5, 9) coming into contact with one another is determined in each of the two functional planes by a joint chain, a dimeric joint chain, with two joint axes running through the centres of curvature $M_1$, $M_2$; $M_3$, $M_4$; $M_{11}$, $M_{22}$; $M_{33}$, $M_{44}$ of the functional surfaces (4, 5; 9, 10) and fixed by these.

2. Artificial joint according to Claim 1, characterized in that the centres of rotation $M_1$ and $M_2$ lie within the joint part having the convex sectional contour, and the joint axis path of the centres of rotation has a radius $RL = R_2 - R_1$, where $R_2$ is greater than $R_1$.

3. Artificial joint according to Claim 1 or 2, characterized in that, in the transverse plane, the lateral functional surface (4) of the lateral joint head (2) and the lateral functional surface (10) of the joint socket (8) have a circular sectional contour, the circular, convex sectional contour of the functional surface (4) having radius $R_{11}$ and centre point $M_{11}$, and the circular, concave functional surface (10) having radius $R_{22}$ and centre point $M_{22}$, and the centres of rotation $M_{11}$ and $M_{22}$ lie in the component with the convex functional surface (4), and the joint axis path of the centres of rotation has a radius $RL_1 = R_{22} - R_{11}$, where $R_{22} > R_{11}$.

4. Artificial joint according to Claim 2 or 3, characterized in that the centre points $M_{11}$ and $M_{22}$ do not coincide with the centres of rotation $M_1$ and $M_2$, and $M_2$ is advantageously offset rearwards in relation to $M_1$, viewed in the extended position of the leg, while $M_{22}$ is offset outwards in relation to $M_{11}$.

5. Artificial joint according to one of Claims 1 to 4, characterized in that the centres of rotation $M_3$ and $M_4$ each lie in the component having the convex sectional contour of the functional surfaces, and the joint axis path of the centres of rotation $M_3$ and $M_4$ has a radius $RM = R_4 - R_3$, where $R_4 > R_3$.

6. Artificial joint according to Claim 5, characterized in that, in the section through the transverse plane, the functional surface (5) of the joint head (3) and the functional surface (9) of the joint socket (7) each have circular sectional contours, the functional surface (5) in this case having a convex sectional contour with centre point $M_{33}$ and radius $R_{31}$, and the functional surface (9) of the medial joint socket having a circular, convex sectional contour with centre of rotation $M_{44}$ and radius $M_{41}$, where the centres of rotation $M_{33}$ and $M_{44}$ are each located within the associated joint component (3, 7) and the joint axis path of the centres of rotation $M_{33}$ and $M_{44}$ has a radius $RM_1 = R_{31} + R_{41}$.

7. Artificial joint according to one of Claims 1 to 6, characterized in that the centre of rotation $M_4$ is offset distally in relation to the centre of rotation $M_3$, i.e. rearwards and downwards, or caudal, and the centre of rotation $M_{44}$ is offset forwards and laterally outwards in relation to the centre of rotation $M_{33}$.

8. Artificial joint according to one of Claims 1 to 7, characterized in that, in the transverse plane or planes, the convex joint heads (2, 3) are connected by an adapted concave structure and in the transverse plane or planes the joint sockets (7, 8) are connected by an adapted convex structure, the radius of the connecting concave structure between the joint heads (2, 3) being advantageously non identical to the radii $R_{44}$ and $R_{22}$, and the radius of the convex structure connecting the joint sockets (7, 8) being greater than that of the concave connecting structure of the joint heads (2, 3).

9. Artificial joint according to one of Claims 1 to 8, characterized in that the centre points $M_1$ and $M_2$ are chosen in relation to one another, and the centre points $M_2$ and $M_4$ are chosen in relation to one another, and the centre points $M_2$ and $M_4$ are chosen in relation to the centre points $M_1$ and $M_2$, in such a way that in the starting position of the standing human knee, the contact points in the lateral joint part and the contact points in the medial joint part lie substantially in an almost horizontal transverse plane.

10. Artificial joint according to one of Claims 1 to 9, characterized in that the joint socket (9, 10) of the patellar joint part (6) has functional surfaces which are each divided into two subsidiary functional surfaces (9a, 9b) and (10a, 10b) lying one above the other, the circular sectional contour of these subsidiary functional surfaces corresponding to that of the corresponding functional surface (9, 10).

11. Artificial joint according to one of Claims 1 to 10, characterized in that the functional surface (9), viewed in the transverse plane, has a concave, circular arc-shaped sectional contour, where the radius of the joint axis path $RM_1 = R_{41} - R_{31}$, with $R_{41} > R_{31}$, the centre points $M_{33}$ and $M_{44}$ lying in the joint component with the convex sectional contour.

## Revendications

1. Articulation artificielle utilisée comme endoprothèse pour l'articulation du genou humain, composée de deux corps d'articulation mobiles l'un par rapport à l'autre, dont un corps d'articulation (1) qui forme la partie de l'articulation côté cuisse et qui possède deux têtes d'articulation regroupées disposées l'une à côté de l'autre, à savoir, une tête d'articulation latérale (2) et une tête d'articulation médiale (3), et un corps d'articulation (6) qui forme la partie d'articulation côté rotule et qui possède deux cuvettes d'articulation regroupées disposées l'une à côté de l'autre, à savoir une cuvette d'articulation latérale (8) et une cuvette d'articulation médiale (7) qui possèdent chacune des surfaces fonctionnelles toroïdales (4, 5 ; 9, 10) qui, dans des plans perpendiculaires entre eux - un plan longitudinal et un plan transversal correspondant - possèdent des profils en coupe circulaires différents, la surface fonctionnelle latérale (4) de la tête d'articulation latérale (2) possédant, dans le plan longitudinal, un profil en coupe en arc de cercle, convexe, de centre de rotation $M_1$ et de rayon $R_1$, et l'articulation médiale possédant, vue dans le plan longitudinal, une tête d'articulation médiale (3) qui possède un profil en coupe circulaire, convexe, de centre de rotation $M_3$ et de rayon $R_3$, et la cuvette d'articulation latérale (8) présentant, vue dans le plan longitudinal, une surface fonctionnelle (10) qui possède un profil en coupe circulaire, concave, de centre de rotation $M_2$ et de rayon $R_2$ tandis que la cuvette d'articulation médiale (7) présente une surface fonctionnelle (9) qui possède, vue dans le plan longitudinal, un profil en coupe circulaire, concave, de centre $M_4$ et de rayon $R_4$, et les rayons $R_1$, $R_2$, $R_3$, $R_4$ étant choisis de manière qu'il soit garanti que les points de contact qui se déplacent autour du centre de rotation $M_1$ décrivent un trajet beaucoup plus grand que les points de contact qui se déplacent autour du centre de rotation $M_3$, et les points de contact représentant chacun les points de contact matériel des surfaces fonctionnelles opposées, et la géométrie d'articulation des surfaces fonctionnelles (4, 10 ; 5, 9) qui entrent en contact étant déterminée, l'une par rapport à l'autre dans chacun des deux plans fonctionnels, par une chaîne d'articulations possédant deux axes d'articulations, des chaînes d'articulation dimères, qui passent par les centres de courbure ($M_1$, $M_2$ ; $M_3$, $M_4$ ; $M_{11}$, $M_{22}$; $M_{33}$, $M_{44}$) des surfaces fonctionnelles (4, 5 ; 9, 10) et sont déterminés par ces dernières.

2. Articulation artificielle selon la revendication 1, caractérisée en ce que les centres de rotation $M_1$ et $M_2$ se trouvent à l'intérieur de la partie d'articulation qui possède le profil en coupe convexe, et la trajectoire d'axe d'articulation des centres de rotation possède un rayon $RL = R_2 - R_1$, où $R_2$ est plus grand que $R_1$.

3. Articulation artificielle selon la revendication 1 ou 2, caractérisée en ce que, dans le plan transversal, la surface fonctionnelle latérale (4) de la tête d'articulation latérale (2) et la surface fonctionnelle latérale (10) de la cuvette d'articulation (8) possèdent un profil en coupe circulaire, le profil en coupe circulaire, convexe de la surface fonctionnelle (4) possédant le rayon $R_{11}$ et le centre $M_{11}$, et la surface fonctionnelle circulaire, concave (10) possédant le rayon $R_{22}$ ainsi que le centre $M_{22}$, et les centres de rotation $M_{11}$ et $M_{22}$ se trouvent dans le corps possédant la surface fonctionnelle convexe (4), et la trajectoire d'axe d'articulation des centres de rotation possède un rayon $RL_1 = R_{22} - R_{11}$, où $R_{22} > R_{11}$.

4. Articulation artificielle selon la revendication 2 ou 3, caractérisée en ce que les centres $M_{11}$ et $M_{22}$ ne coïncident pas avec les centres de rotation $M_1$ et $M_2$ respectivement, et, dans la position de la jambe étendue, $M_2$ est avantageusement déporté vers l'arrière par rapport à $M_1$, tandis que $M_{22}$ est déporté vers l'extérieur par rapport à $M_{11}$.

5. Articulation artificielle selon une des revendications 1 à 4, caractérisée en ce que les centres de rotation $M_3$ et $M_4$ se trouvent chacun dans le corps possédant le profil en coupe convexe des surfaces fonctionnelles, et la trajectoire d'axe d'articulation des centres de rotation $M_3$ et $M_4$ présente un rayon $RM = R_4 - R_3$, où $R_4$ est $> R_3$.

6. Articulation artificielle selon la revendication 5, caractérisée en ce qu'en coupe dans le plan transversal, la surface fonctionnelle (5) de la tête d'articulation (3) et la surface fonctionnelle (9) de la cuvette d'articulation (7) présentent chacune un profil en coupe circulaire, la surface fonctionnelle (5) possédant ici un profil en coupe convexe de centre $M_{33}$ et de rayon $R_{31}$ et la surface fonctionnelle (9) de la cuvette d'articulation médiale possédant un profil en coupe circulaire, convexe, de centre de rotation $M_{44}$ et de rayon $R_{41}$, les centres de rotation $M_{33}$ et $M_{44}$ se trouvant chacun à l'intérieur du corps d'articulation correspondant (3, 7) et la trajectoire d'axe d'articulation des centres de rotation $M_{33}$ et $M_{44}$ possédant un rayon $RM_1 = R_{31} + R_{41}$.

7. Articulation artificielle selon une des revendications 1 à 6, caractérisée en ce que par rapport au centre de rotation $M_3$, le centre de rotation $M_4$ est déporté dans la direction distale, c'est-à-dire vers l'arrière et vers le bas, dans la direction caudale, tandis que, par rapport au centre de rotation $M_{33}$, le centre de rotation $M_{44}$ est déporté vers l'avant et vers l'extérieur, dans la direction latérale.

8. Articulation artificielle selon une des revendications 1 à 7, caractérisée en ce que, dans le plan transversal ou les plans transversaux, les têtes d'articulation convexes (2, 3) sont reliées par une structure concave accordée et, dans le plan transversal ou les plans transversaux, les cuvettes d'articulation (7, 8) sont reliées par une structure convexe accordée, et, dans un mode avantageux, le rayon de la structure concave de liaison entre les têtes d'articulation (2, 3) n'est pas identique aux rayons $R_{44}$ et $R_{22}$, et que le rayon de la structure convexe qui relie les cuvettes d'articulation (7, 8) est plus grand que celui de la structure concave de liaison des têtes d'articulation (2, 3).

9. Articulation artificielle selon une des revendications 1 à 8, caractérisée en ce que les centres $M_1$ et $M_2$, l'un par rapport à l'autre, et les centres $M_2$ et $M_4$, l'un par rapport à l'autre, de même que les centres $M_2$ et $M_4$ par rapport aux centres $M_1$ et $M_2$, sont choisis de manière que, dans la position de départ de la position debout du genou humain, les points de contact dans la partie latérale comme dans la partie médiale de l'articulation se trouvent dans une large mesure dans un plan transversal presque horizontal.

10. Articulation artificielle selon une des revendications 1 à 9, caractérisée en ce que la cuvette d'articulation (9, 10) de la partie d'articulation côté rotule (6) présente des surfaces fonctionnelles qui sont divisées chacune en deux surfaces fonctionnelles partielles (9a, 9b) et (10a, 10b) placées l'une au-dessus de l'autre, le profil en coupe circulaire de ces surfaces fonctionnelles partielles correspondant à celui de la surface fonctionnelle correspondante (9, 10).

11. Articulation artificielle selon une des revendications 1 à 10, caractérisée en ce que, vue dans un plan transversal, la surface fonctionnelle (9) possède un profil en coupe concave, en forme d'arc de cercle, le rayon de la trajectoire d'axe d'articulation est $RM_1 = R_{41} - R_{31}$, avec $R_{41} > R_{31}$, les centres $M_{33}$ et $M_{44}$ se trouvant à l'intérieur du corps d'articulation qui possède le profil en coupe convexe.

FIG.1

3 → 2

5 4 → 1

6

8 7

10 9

FIG.2

FIG.3

FIG. 4

FIG.5

FIG.6

**FIG.7**

**FIG.8**